(19) **Europäisches Patentamt / European Patent Office / Office européen des brevets**

(11) **EP 1 916 295 A2**

(12) **EUROPEAN PATENT APPLICATION**

(43) Date of publication:
**30.04.2008 Bulletin 2008/18**

(51) Int Cl.:
*C11D 3/02* (2006.01)　　　*A61K 8/96* (2006.01)
*A61Q 5/02* (2006.01)　　　*A61Q 19/10* (2006.01)

(21) Application number: **07119023.5**

(22) Date of filing: **22.10.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **25.10.2006 JP 2006290413**

(71) Applicant: **MIURA CO., LTD.**
**Matsuyama-shi**
**Ehime 799-2696 (JP)**

(72) Inventors:
  • **Yoshinari, Yuji**
  **Matsuyama-shi Ehime 799-2696 (JP)**

  • **Takai, Masaki**
  **Matsuyama-shi Ehime 799-2696 (JP)**
  • **Hatori, Makoto**
  **Matsuyama-shi Ehime 799-2696 (JP)**
  • **Yamamoto, Daisuke**
  **Matsuyama-shi Ehime 799-2696 (JP)**
  • **Okamoto, Yuki**
  **Matsuyama-shi Ehime 799-2696 (JP)**

(74) Representative: **Marchant, James Ian**
  **Elkington and Fife LLP**
  **Prospect House**
  **8 Pembroke Road**
  **Sevenoaks, Kent TN13 1XR (GB)**

(54) **Cleaning Agent**

(57)    The cleaning agent, which is less likely to retain a surfactant on an object to be cleaned and not to restrict applicable objects to be cleaned, contains water from which polyvalent cations are removed and to which a sodium ion is added, and a surfactant. An example of the surfactant is a fatty acid salt. When the cleaning agent is applied to an object to be cleaned, stain adhered on the object is removed from the object by action of the surfactant. The surfactant hardly remains on the object due to the action of the water from which polyvalent cations are removed and to which a sodium ion is added. Accordingly, the cleaning agent can effectively wash a wide variety of objects, such as kitchens, tableware, food, washstands, bathrooms, toilets, vehicles, clothes and body skin, without damaging texture and deteriorating quality.

**EP 1 916 295 A2**

**Description**

Technical Field

The present invention relates to a cleaning agent, in particular, a cleaning agent using a surfactant.

Background Art

In cleaning kitchen sinks, tableware, sanitary installations such as bathrooms and toilets, clothes, vehicles, food, bodies and the like, a surfactant is commonly used. In the cleaning using a surfactant, in general, a surfactant is absorbed in a cleaning tool such as a cloth and a sponge, and is foamed to rub an object to be cleaned, followed by rinsing the object with water. Stain adhered onto the object comes onto a surface by the action of a surfactant, and therefore it is rinsed off from the object upon rinsing with water.

However, a surfactant applied to an object to be cleaned is apt to remain on the object, even if it is rinsed off carefully with water. The residual surfactant on the object forms a film on a surface of the object, and which may not only damage surface texture of the object, such as luster, but also there is a possibility of exerting an adverse effect such as deterioration of the object. In addition, since a surfactant becomes a source of nutrient for fungi and bacteria, parasitism and propagation of fungi and bacteria can easily occur in the object with residual surfactant therein.

Given this, various countermeasures have been studied in order to suppress the remnant of a surfactant. For example, Japanese Unexamined Patent Publication (Kohyo) No. 10-508901 (JP 1998-508901 A) discloses a cleaning fluid, which comprises a surfactant and a hydrotrope compound such as benzene sulfonate and naphthalene sulfonate dissolved in water. In the cleaning fluid, the hydrotrope compound suppresses a surfactant forming a film on a surface of an object to be cleaned. As a result, the surfactant hardly remains on the object after cleaning.

However, when the effect that the hydrotrope compound exerts on food and human bodies is considered, applicability of the cleaning fluid is restricted to hard objects such as glass and ceramic tiles. Furthermore, when an object to be cleaned requires sterilization, the cleaning fluid needs to further contain a quaternary ammonium compound, too.

An object of the present invention is to realize a cleaning agent, which is less likely to retain a surfactant on an object to be cleaned and not to restrict applicable objects to be cleaned.

Summary of the Invention

A cleaning agent of the present invention contains water, from which polyvalent cations are removed and to which a sodium ion is added, and a surfactant.

When the cleaning agent is applied to an object to be cleaned, stain adhered onto the object is removed therefrom by the action of the surfactant. The surfactant hardly remains on the object due to the action of the water, from which polyvalent cations are removed and to which sodium ions are added. Accordingly, the cleaning agent washes an object effectively, without damaging texture and deteriorating the quality of the object.

For this reason, the cleaning agent is not likely to restrict applicable objects to be cleaned, and can be used for a wide variety of applications such as kitchens, tableware, foods, washstands, bathrooms, toilets, vehicles, clothes and human skin.

The surfactant used in the cleaning agent of the present invention is, for example, a fatty acid salt. Particularly, unsaturated fatty acid salts are preferable. Examples of the unsaturated fatty acid salts include at least one kind selected from the group consisting of linoleic acid, linolenic acid, myristoleic acid and palmitoleic acid.

Other objects and effects of the present invention will be described in detail hereinafter.

Brief Description of the Drawings

Fig. 1 is a graph showing a result of cleaning efficiency of a stained cloth 1, with regard to Evaluation 4 of Examples.
Fig. 2 is a graph showing a result of cleaning efficiency of a stained cloth 2, with regard to Evaluation 4 of Examples.
Fig. 3 is a graph showing a result of cleaning efficiency of a stained cloth 3, with regard to Evaluation 4 of Examples.
Fig. 4 is a graph showing a result of cleaning efficiency of a stained cloth 4, with regard to Evaluation 4 of Examples.
Fig. 5 is a graph showing a result of Evaluation 5 of Examples.
Fig. 6 is a graph showing results of Examples 17 to 25.
Fig. 7 is a graph showing results of Examples 26 and 27.
Fig. 8 is a graph showing results of Examples 28 and 29.

Fig. 9 is a graph showing results of Examples 30 and 31.
Fig. 10 is a graph showing results of Examples 32 and 33 and Comparative Examples 13 and 14.
Fig. 11 is a graph showing a result of measuring stratum corneum moisture content in Evaluation A of Example 34.
Fig. 12 is a graph showing a result of measuring skin elasticity in Evaluation A of Example 34.
Fig. 13 is a graph showing a result of measuring texture density in Evaluation A of Example 34.

Description of the Preferred Embodiments

The cleaning agent of the present invention contains water from which polyvalent cations are removed and to which sodium ions are added (hereafter such water is called "functional water" in some cases), and a surfactant.

The functional water used in the present invention is obtained by treating water (raw water) such as tap, ground, river, lake and well water, with cation exchange resin. In this treatment, a calcium ion (bivalent cation), magnesium ion (bivalent cation), copper ion (bivalent cation), iron ion (bivalent and trivalent cations), aluminum ion (trivalent cation) and the like, that are contained in the raw water are exchanged, with a sodium ion (monovalent cation) contained in the cation exchange resin.

The cation exchange resin used for the treatment of raw water is a synthetic resin, wherein a suflonic acid group is introduced to a matrix of a cross-linked three-dimensional polymer such as a copolymer of styrene and divinylbenzene, and the sulfonic acid group forms a sodium salt.

In the functional water, it is preferable that a concentration of polyvalent cations is commonly adjusted to less than 0.2 mmol/l, and particularly preferable to be adjusted to less than the measurement limit, which signifies substantially zero level. Here, the concentration of polyvalent cations denotes a concentration measured on the basis of ICP emission spectroscopic analysis.

On the other hand, in the functional water, it is preferable that a concentration of a sodium ion is commonly adjusted to 0.3 mmol/l or more and less than 500 mmol/l, and more preferable to be adjusted to 0.5 mmol/l or more and less than 200 mmol/l. Here, the concentration of a sodium ion denotes a concentration measured on the basis of ICP emission spectroscopic analysis.

A surfactant used in the present invention is not particularly limited. Examples thereof include anionic, cationic, ampholytic and nonionic surfactants.

Examples of an anionic surfactant include fatty acid salts (soaps), alkylbenzene sulfonate salts, alkyl sulfate salts, $\alpha$-olefin sulfonate salts and N-acyl glutamate salts. Two or more of these anionic surfactants may be used in combination.

Examples of a cationic surfactant include N-alkyltrimethyl ammonium chloride and N-alkylbenzyl dimethyl ammonium chloride. Two or more of these cationic surfactants may be used in combination.

Examples of an ampholytic surfactant include N-alkyl-$\beta$-alanine and N-alkylcarboxy betaine. Two or more of these ampholytic surfactants may be used in combination.

Examples of a nonionic surfactant include polyoxyethylene alkyl ether, polyoxyethylene alkylphenyl ether, fatty acid diethanol amide and fatty acid sucrose ester. Two or more of these nonionic surfactants may be used in combination.

In the present invention, the above various surfactants can be used in combination with other kinds of surfactants.

Preferred surfactants used in the present invention are fatty acid salts, because they have effective sterilization action to an object to be cleaned. Particularly alkali metal salts of saturated or unsaturated fatty acid having 5 to 22 carbon atoms are preferred. An alkali metal salt of saturated fatty acid and an alkali metal salt of unsaturated fatty acid can be used in combination.

With regard to the saturated fatty acid salts, those having 12 to 16 carbon atoms are particularly preferable. Specifically, sodium salts and potassium salts of lauric acid, myristic acid, pentadecylic acid and palmitic acid are exemplified. On the other hand, with regard to the unsaturated fatty acid salts, those having 14 to 18 carbon atoms are preferred, and those having a larger number of unsaturated bonds between carbons are particularly preferred. Specific examples of the unsaturated fatty acids include sodium salts and potassium salts of myristoleic acid, palmitoleic acid, oleic acid, linoleic acid and linolenic acid.

As for fatty acid salts, it is preferable to use unsaturated fatty acid salts because of their exhibiting high sterilizing power to an object to be cleaned. Particularly, salts of linoleic acid, linolenic acid, myristoleic acid and palmitoleic acid are preferred, and sodium salts thereof are more preferred.

In the cleaning agent of the present invention, an amount of a surfactant to be used is preferably adjusted to from 10 mg to 400 g per liter of functional water, and more preferably from 20 mg to 200 g. When the amount of the surfactant is less than 10 mg, there is a possibility that the cleaning agent of the present invention does not exhibit effective sterilization action. On the contrary, when it exceeds 400 g, the surfactant is apt to remain in an object to be cleaned, and there is a possibility that the residual surfactant damages texture of the object, or deteriorate the quality thereof. In addition, there is a possibility that the residual surfactant becomes a source of nutrient for fungi and bacteria and causes propagation thereof in the object.

The cleaning agent of the present invention may contain some other components other than the above-described functional water and surfactant as long as the object of the present invention is not adversely affected. Examples of other components include fragrant materials such as grapefruit oil, spearmint oil, nutmeg oil and mandarin oil, and antioxidants such as tocopherol, ascorbyl stearate ester, sodium erythorbate, ascorbic acid, citric acid and dibutyl hydroxytoluene. Two or more of the fragrant materials and antioxidants can be used in combination.

The cleaning agent of the present invention can be easily prepared through the processes of treating raw water with the above cation exchange resin, and to the resulting functional water, properly adding a surfactant and, if necessary, the above other components. Accordingly, the cleaning agent is easily produced in large quantity, and also can be produced at low cost.

An object to which the cleaning agent of the present invention can be applied is not particularly limited. Examples thereof include kitchens (such as sinks, floors and walls), tableware (such as glass ware, earthenware, porcelain, metallic ware, chopsticks and cutlery), food such as vegetables and fruits, washstands, bathrooms (such as bathtubs, floors, walls, drain outlets and plated parts such as faucets), toilets (such as toilet bowls, floors and walls), tubs of washing machines, vehicles (such as automobiles, two-wheeled motor vehicles and railroad vehicles), clothes and daily commodities (such as rainwear, footwear and linens).

When the above objects, particularly kitchens, tableware, washstands, bathrooms, toilets, tabs of washing machines, vehicles and daily commodities such as rainwear, are washed with the cleaning agent of the present invention, in general, the cleaning agent is absorbed in a cleaning tool such as a kitchen cloth, sponge or brush, foamed to wipe or rub an object to be cleaned with the cleaning tool, and rinsed off with water. In rinsing the object, it is preferable to use functional water only. The object after rinsed with water can be dried as it is, but can be wiped with cloth or paper to remove water.

When food is washed, the food is immersed in the cleaning agent and washed, and then rinsed with water, preferably with functional water.

Further, in case of washing water-absorbing objects such as daily commodities, for example, clothes, footwear and linens, in general, the object is immersed in the cleaning agent of the present invention and squeeze washed therein, and then rinsed with water, preferably with the functional water. The cleaning operation of this kind may be carried out manually or, according to a kind of the object, performed by a washing machine.

The cleaning agent of the present invention can also be used for washing body skin. In this case, the cleaning agent is absorbed in a body washer tool such as a cloth, sponge or brush, foamed to wipe or rub skin with the body washer tool, and rinsed off with water. It is preferable to use functional water only to rinse the skin. When the cleaning agent of the present invention is used for washing hands, a proper amount of the cleaning agent can be directly taken in the hands, the hands are rubbed together to wash, and then rinsed off with water (preferably with functional water only).

In the object cleaned with the cleaning agent of the present invention, stain adhered thereto is removed by the action of a surfactant. The surfactant is not likely to remain on the object by action of the functional water. Particularly, when the object after washing is rinsed with the functional water only, the surfactant is effectively rinsed away from the object and hardly remains thereon. Consequently, in the object after cleaning, change or deterioration of texture, such as luster and sense of touch, caused by the effect of a surfactant is hard to occur.

An object washed with the cleaning agent of the present invention is hygienic because it is sterilized upon washing by the action of the surfactant. In addition, the hygienic state tends to be maintained because the surfactant is hard to remain as described above, which otherwise can be a source of nutrient for fungi and bacteria, and thus parasitism or propagation of fungi and bacteria is suppressed.

In the above embodiment, water from which polyvalent cations are removed and to which a sodium ion is added is used as functional water, but the functional water may be such that polyvalent cations are removed and an alkali metal ion other than a sodium ion, such as a potassium ion, is added. Functional water of this kind can be obtained by treating raw water with a cation exchange resin, wherein sulfonic acid group forms an alkali metal salt such as a potassium salt.

Examples

Examples 1 to 6 and Comparative Examples 1 to 6

A test piece, all over which a model contamination solution was adhered, was fully immersed in a cleaning agent at 30°C, and then allowed to stand for 10 minutes. The test pieces and cleaning agents used herein are as follows, and combinations of the test piece and the cleaning agent are shown in Table 1.

[Test pieces]

<Test piece 1>

A rectangular plate material (76 mm × 26 mm × 1.0 mm) made of borosilicate glass was immersed in a model contamination solution prepared by adding a red colorant (Sudan III) to a mixture of beef tallow and soybean oil, and thereby the model contamination solution was adhered onto the entire surface of the plate material.

<Test piece 2>

A rectangular plate material (76 mm × 26 mm × 1.0 mm) made of borosilicate glass was immersed in a model contamination solution prepared by dissolving gelatin in water, and thereby the model contamination solution was adhered onto the entire surface of the plate material.

<Test piece 3>

A rectangular plate material (76 mm × 26 mm × 1.0 mm) made of borosilicate glass was immersed in a model contamination solution prepared by dissolving albumin in water, and thereby the model contamination solution was adhered onto the entire surface of the plate material.

[Cleaning agents]

<Cleaning agent 1>

A cleaning agent, which is prepared by adding and dissolving 0.8 g of a soap (trade name of "Nantaro Powder Soap" manufactured by Miura Co., Ltd.) per liter of functional water obtained by treating tap water supplied in Matsuyama City, Ehime Japan, with a cation exchange resin. The functional water satisfies the following conditions: a concentration of polyvalent cations is less than 0.2 mmol/l; and a concentration of a sodium ion is 0.3 mmol/l or more and less than 500 mmol/l.

<Cleaning agent 2>

A cleaning agent, which is prepared by adding and dissolving 0.8 g of a soap (trade name of "Nantaro Powder Soap" manufactured by Miura Co., Ltd.) per liter of tap water supplied in Matsuyama City, Ehime Japan.

<Cleaning agent 3>

A cleaning agent, which is prepared by adding and dissolving a 0.75 ml of synthetic detergent (trade name of "Family Compact" manufactured by Kao Corporation) per liter of the functional water that was used in the preparation of the cleaning agent 1.

<Cleaning agent 4>

A cleaning agent, which is prepared by adding and dissolving 0.75 ml of a synthetic detergent (trade name of "Family Compact" manufactured by Kao Corporation) per liter of tap water supplied in Matsuyama City, Ehime Japan.

Evaluation 1

In Examples 1 to 6 and Comparative Examples 1 to 6, a test piece was taken out of a cleaning agent 10 minutes after immersion had started, and a cleaning ratio of the test piece was determined. The cleaning ratio was determined by the following method. The results are shown in Table 1.

[Cleaning ratio of Test piece 1]

A mixture of beef tallow and soybean oil adhered to the test piece was extracted in chloroform, and an amount of the mixture contained in the extraction solution was determined by absorption spectrophotometry (510 nm) . The cleaning ratio (%) was calculated by the formula: (A - B)/A × 100 (wherein A represents the amount of mixture adhered to the test piece before washing; and B represents the amount of mixture contained in the extraction solution).

[Cleaning ratio of Test piece 2]

The test piece was immersed in an aqueous solution of NaOH (0.1 N) at 85 $\pm$ 5°C, and treated for 120 minutes. Then, the amount of gelatin contained in the NaOH aqueous solution was determined by absorption spectrophotometry (562 nm) using BCA Protein Assay Kit manufactured by Pierce Chemical Company. The cleaning ratio (%) was calculated by the formula: (A - B)/A $\times$ 100 (wherein A represents the amount of gelatin adhering to the test piece before washing; and B represents the amount of gelatin contained in the NaOH aqueous solution).

[Cleaning ratio of Test piece 3]

The test piece was immersed in an aqueous solution of NaOH (0.1 N) at 85 $\pm$ 5°C, and treated for 120 minutes. Then, the amount of albumin contained in the NaOH aqueous solution was determined by absorption spectrophotometry (562 nm) using BCA Protein Assay Kit manufactured by Pierce Chemical Company. The cleaning ratio (%) was calculated by the formula: (A - B)/A $\times$ 100 (wherein A represents the amount of albumin adhering to the test piece before washing; and B represents the amount of albumin contained in the NaOH aqueous solution).

Table 1

|  | Test piece | Cleaning agent | Cleaning Ratio (%) |
|---|---|---|---|
| Example 1 | 1 | 1 | 99.4 |
| Comparative Example 1 | 1 | 2 | 21.9 |
| Example 2 | 1 | 3 | 99.7 |
| Comparative Example 2 | 1 | 4 | 99.5 |
| Example 3 | 2 | 1 | 96.9 |
| Comparative Example 3 | 2 | 2 | 94.6 |
| Example 4 | 2 | 3 | 90.6 |
| Comparative Example 4 | 2 | 4 | 90.1 |
| Example 5 | 3 | 1 | 99.8 |
| Comparative Example 5 | 3 | 2 | 99.1 |
| Example 6 | 3 | 3 | 99.9 |
| Comparative Example 6 | 3 | 4 | 95.1 |

Example 7

In accordance with "JEMA-HD84, A method for performance measurement of dishes washing/drying machine", which is a voluntary standard stipulated by the Japan Electrical Manufacturer's Association, a group of stained tableware (total number of stained tableware = 56) were prepared with the content below. After leaving the stained tableware for 1 hour, they were washed through selecting a "standard course" program of an automatic dishes washing/drying machine (trade name of "NP-40SX2" manufactured by Matsushita Electric Industrial Co., Ltd.). The functional water (satisfying the following conditions: a concentration of polyvalent cations is less than 0.2 mmol/l; and a concentration of sodium ions is 0.3 mmol/l or more and less than 500 mmol/l) obtained by treating tap water supplied in Matsuyama City, Ehime Japan, with a cation exchange resin was supplied to the automatic dishes washing/drying machine as a cleaning water. The amount of a detergent (trade name of "Hiwash A" manufactured by Procter & Gamble Japan) to be used was determined to 5 g.

A group of stained tableware

| Tableware | Number | State of stain |
|---|---|---|
| Large plate | 4 pieces | Spread a mixture of curried rice and raw egg, and leave about 10 rice grains over the plate |

(continued)

| Tableware | Number | State of stain |
|---|---|---|
| Middle plate | 2 pieces | Chop up pork cutlet with pork cutlet sauce thereover, and spread it over the plate |
| Small plate | 4 pieces | Chop up a half-cooked fried egg, and spread it over the plate |
| Rice bowl | 6 pieces | Spread rice in the bowl |
| Soup bowl | 6 pieces | Rinse the bowl with miso soup |
| Teacup | 4 pieces | Rinse the cup with green tea |
| Glass | 3 pieces | Rinse the glass with tomato juice |
| Glass | 3 pieces | Rinse the glass with milk |
| Chopsticks | 12 pairs | Stained at the time when the rice bowls were stained, and adhere a rice grain on the tip of the chopstick |
| Fork | 4 pieces | Stained at the time when the middle and small plates were stained |
| Spoon | 4 pieces | Stained at the time when the large plates were stained |
| Knife | 4 pieces | Stained at the time when the middle and small plates were stained |

Comparative Example 7

A group of stained tableware was washed in the same manner as in Example 7, except for using tap water supplied in Matsuyama City, Ehime Japan, as a cleaning water.

Example 8

A group of stained tableware was washed in the same manner as in Example 7, except for changing an amount of the detergent to 10 g.

Comparative Example 8

A group of stained tableware was washed in the same manner as in Comparative Example 7, except for changing the amount of the detergent to 10 g.

Evaluation 2

With regard to Examples 7 and 8 and Comparative Examples 7 and 8, in accordance with "JEMA-HD84, A method for performance measurement of dishes washing/drying machine", which is a voluntary standard stipulated by the Japan Electrical Manufacturer's Association, the finishing state of the group of the stained tableware after washing was evaluated based on the criteria below, and a cleaning ratio was calculated by the following equation (1). In the equation (1), "Number" denotes the number of relevant stained tableware, and "Total number" denotes a total number of stained tableware. The results are shown in Table 2.

Rate A: Cleaned to the extent that no stain adherence is visually observed, and there is no region of oil film and cloud.

Rate B: Cleaned to the extent that the tableware can be used without washing again, and the state of stain adherence and cloud is such extent that is described in (a) and (b).

(a) A number of regions where a stain adheres is 4 or less, and also a total adherence area of a stain is 4 mm$^2$ or less.
(b) A total cloud area is 1 cm$^2$ or less.

Rate C: Cleaned to the extent that neither rank A nor rank B is gained.

$$\text{Cleanig ratio (\%)} = \frac{(\text{Number of rate A}) \times 2 + (\text{Number of rate B})}{\text{Total number} \times 2} \times 100$$

$$(1)$$

Table 2

| | Finishing state (number) | | | Cleaning ratio (%) |
|---|---|---|---|---|
| | Rate A | Rate B | Rate C | |
| Example 7 | 17 | 24 | 15 | 52 |
| Comparative Example 7 | 16 | 10 | 30 | 38 |
| Example 8 | 19 | 21 | 16 | 53 |
| Comparative Example 8 | 19 | 9 | 28 | 42 |

Example 9

By a "standard course" program of an automatic dishes washing/drying machine (trade name of "NP-40SX2" manufactured by Matsushita Electric Industrial Co., Ltd.), one piece of knife (made of 18-8 stainless), one pair of chopsticks (made of wood coated with urethane) and one piece of large plate (white ceramic made of quartz glass) were washed. The knife and other tableware washed herein were those without stain. The functional water (satisfying the following conditions: a concentration of polyvalent cations is less than 0.2 mmol/l; and a concentration of sodium ions is 0.3 mmol/l or more and less than 500 mmol/l) obtained by treating tap water supplied in Matsuyama City, Ehime Japan, with a cation exchange resin was supplied to the automatic dishes washing/drying machine as a cleaning water. The amount of a detergent containing a nonionic surfactant (trade name of "Hiwash A" manufactured by Proctor & Gamble Japan) to be used is determined to 5 g. A temperature of water was adjusted at 30 to 80°C, and washing time was determined as about 2 hours.

Comparative Example 9

A knife, a pair of chopsticks and a large plate were washed in the same manner as in Example 9, except for using tap water of Matsuyama City, Ehime Japan, instead of the functional water.

Evaluation 3

In Example 9 and Comparative Example 9, residual amounts of a nonionic surfactant remaining on the knife, chopsticks and large plate after washing were analyzed. The results are shown in Table 3. The results shown in Table 3 are mean values when the same test was repeated 3 to 5 times. A residual amount of the nonionic surfactant was measured as follows. First, the knife, chopsticks and large plate after washing were immersed in 100% by weight of methanol to dissolve the residual nonionic surfactant. Next, the methanol solution was concentrated by an evaporator, and the concentrate was diluted with distilled water so as to adjust the concentration of methanol to 10% by weight. The color of the resulting methanol solution diluted in this way was developed by using an agent with a trade name of "Nonionic surfactant AE ELISA Kit" manufactured by Takeda Pharmaceutical Co., Ltd., and the absorbance was measured using a spectrophotometer (trade name of "UV-1600PC", measurement wavelength: 450 nm) manufactured by Shimazu Corp. Based on the measurement values, a concentration of the nonionic surfactant was evaluated.

Table 3

| | Residual amount of nonionic surfactant ($\mu$g/cm$^2$) | | |
|---|---|---|---|
| | Knife (surface area: 135 cm$^3$) | Chopsticks (surface area: 45 cm$^3$) | Large plate (surface area: 508 cm$^3$) |
| Example 9 | 0.0002 | 0.08 | 0.00008 |
| Comparative Example 9 | 0.01 | 0.2 | 0.0016 |

Example 10

Using a drum-type home washing machine (trade name of "TW-742EX" manufactured by Toshiba Corporation), 32 pieces of clothes consisting of 4 different kinds of artificially stained clothes of 8 pieces and 3.5 kg of laundry in total (sheet, bath towel, face towel and yukata (cotton wear)) were washed simultaneously. The washing was conducted using a detergent under conditions of several different detergent usage rates. The detergent used herein is that with a trade name of "Ecomax Liquid KW" manufactured by Nicca Chemical Co., Ltd., and an alkaline agent (sodium metasilicate nonahydrate manufactured by Wako Pure Chemical Industries Ltd.) and a bleaching agent (trade name of "Lipo Bleach HP" manufactured by Nicca Chemical Co., Ltd.) were mixed therewith according to the prescription described in the instruction manual of the detergent. The detergent usage rate is calculated by the following equation (2). In the equation (2), "Detergent dosage instructed by product" denotes the usage amount of the detergent prescribed in the instruction manual of the detergent as stated above.

$$\text{Detergent usage rate (\%)} = \frac{\text{Detergent dosage}}{\text{Detergent dosage instructed by product}} \times 100$$

$$(2)$$

The washing machine was programmed such that a washing process, a first rinsing process, a second rinsing process, a third rinsing process, a spin-drying process and a drying up process were conducted in this order. In the washing process and respective rinsing processes, the functional water (satisfying the following conditions: a concentration of polyvalent cations is less than 0.2 mmol/l; and a concentration of sodium ions is 0.3 mmol/l or more and less than 500 mmol/l) obtained by treating tap water supplied in Matsuyama City, Ehime Japan, with a cation exchange resin was supplied. In the washing process, the functional water was adjusted at a temperature of 60°C.
The stained clothes used herein are as follows.

<Stained Cloth 1>

A wet-type artificially stained cloth serving as a model of dirty collar. Specifically, it is described in the Japanese Industrial Standards JIS C 9606 "Detergency test of electric washing machine".

<Stained Cloth 2>

An artificially stained cloth manufactured by EMPA (trade name of "EMPA101"), which is a model cloth stained with a mixture of olive oil and carbon black.

<Stained Cloth 3>

An artificially stained cloth manufactured by EMPA (trade name of "EMPA111"), which is a model cloth stained with blood.

<Stained Cloth 4>

An artificially stained cloth manufactured by EMPA (trade name of "EMPA112"), which is a model cloth stained with a mixture of cocoa powder, sugar and milk.

Comparative Example 10

A washing was conducted in the same manner as in Example 10, except for using tap water of Matsuyama City, Ehime Japan, instead of the functional water in the washing process and the respective rinsing processes.

Evaluation 4

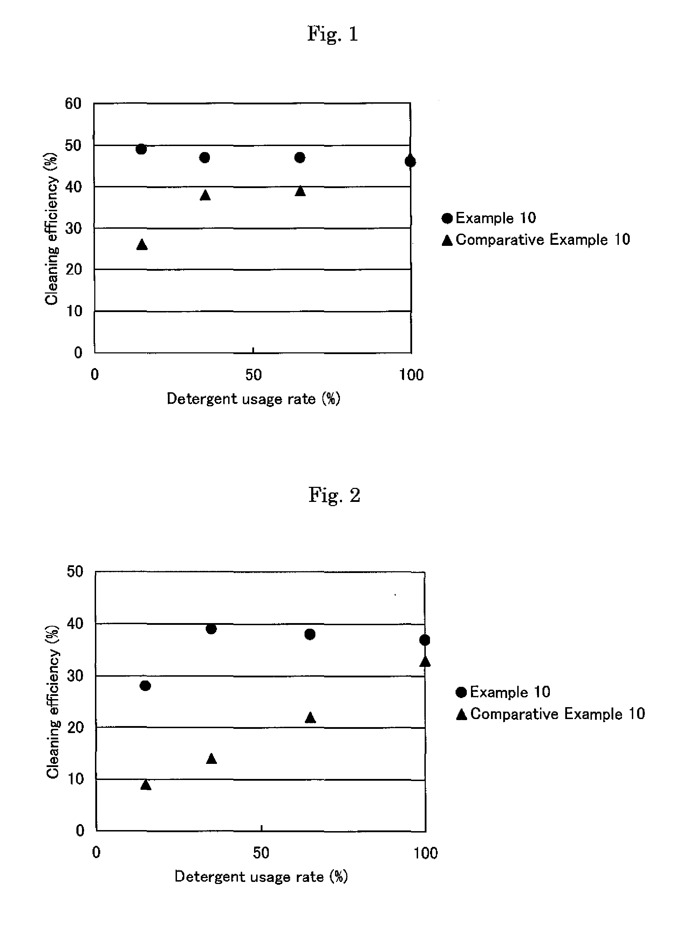

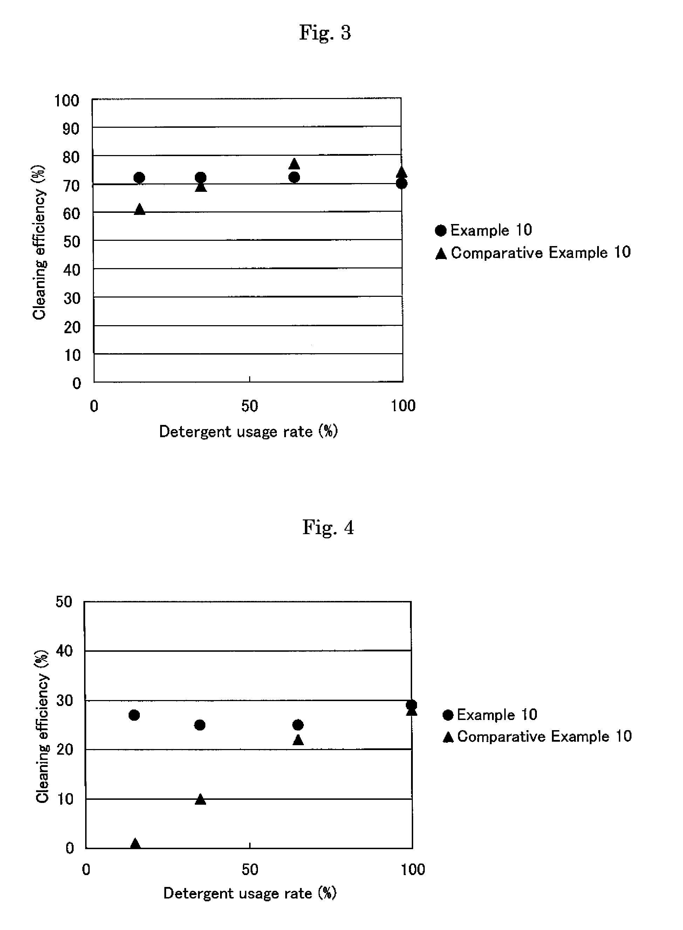

In Example 10 and Comparative Example 10, cleaning efficiency of the respective stained clothes was studied after washing. The cleaning efficiency of the stained cloth 1 was calculated by the following equation (3), and that of the stained clothes 2 to 4 was calculated by the following equation (4). The result of the cleaning efficiency of the stain cloth 1 is shown in Fig. 1, that of the stain cloth 2 is shown in Fig. 2, that of the stain cloth 3 is shown in Fig. 3, and that of the stain cloth 4 is shown in Fig. 4, respectively. The cleaning efficiency shown in each Fig. is a mean value of 8 pieces of respective stained clothes.

$$\text{Cleaning efficiency (\%)} = \frac{\text{Reflectance after washing (\%)} - \text{Reflectance before washing (\%)}}{\text{Reflectance of white cloth (\%)} - \text{Reflectance of before washing (\%)}} \times 100$$

$$(3)$$

In the equation (3), the white cloth is a cotton cloth for cleaning test designated by the Japan Oil Chemists' Society. Reflectance denotes a reflectance at 530 nm. The reflectance was determined by using a reflectometer (trade name of "Spectroscopic Colorimeter SE2000" manufactured by Nippon Denshoku Industries).

$$\text{Cleaning efficiency (\%)} = \frac{\text{Y-value after washing} - \text{Y-value before washing}}{\text{Y-value of white cloth} - \text{Y-value before washing}} \times 100$$

$$(4)$$

In the equation (4), the white cloth is identical to that in the equation (3). The Y-value denotes a Y-value of tristimulus value (that is, brightness of color). The Y-value was determined by using the above reflectometer.
According to Figs. 1 to 4, the cleaning efficiency to be obtained under use conditions that the detergent product intends (that is, a detergent usage rate of 100% in Comparative Example 10) is achieved in Example 10 under a detergent usage rate of 50% or less. This enables to wash laundry efficiently while suppressing a dosage of the detergent.

Examples 11 to 14

The residual state of a surfactant was studied on 3 examinees by applying a cleaning agent shown in Table 4 to antebrachial skin of their elbow (hereinafter called "test region"). Herein, the test region was defatted using ethanol, washed with pure water and then immersed in a cleaning agent for 5 minutes. Next, a rinsing water of 37°C shown in Table 4 was watered to run off over the test region at a flow rate of 3.3 1/min for 36 seconds, and the test region was air-dried. Then, a tape (trade name of "Cellophane Tape" manufactured by Nitto Denko Corp.) was attached onto the test region, and was removed. The components adhered onto the tape were extracted in ether to prepare a sample for analysis. The sample was analyzed by gas chromatography, and an amount of the surfactant adhered onto the tape was quantified. The results are shown in Table 4.
The cleaning agents and rinsing waters shown in Table 4 are as follows.

(Cleaning agent)

A:

A solid soap (trade name of "Shokubutsu Monogatari; makeup soap" manufactured by Lion Corp.), which comprises 83.1% by weight of soap components (sum of 16.6% by weight of sodium laurate, 28.7% by weight of sodium palmitate, 25.0% by weight of sodium oleate and 12.8% by weight of other fatty acid sodium) and 16. 9% by weight of components other than the soap components, was dissolved in functional water so as a concentration to be 5% by weight. The functional water was prepared by treating tap water supplied in Matsuyama City, Ehime Japan, with a cation exchange resin, and satisfied the following conditions: a concentration of polyvalent cations is less than 0.2 mmol/l; and a concentration of sodium ions is 0.3 mmol/l or more and less than 500 mmol/l.

B:

A liquid soap (trade name of "Shokubutsu Monogatari; body soap" manufactured by Lion Corp.), which comprises 18.8% by weight of soap components (sum of 8.5% by weight of sodium laurate and 10.3% by weight of sodium myristate) and 81.2% by weight of components other than the soap components, was dissolved in functional water so as a concentration to be 14% by volume. The functional water was prepared by treating tap water supplied in Matsuyama City, Ehime Japan, with a cation exchange resin, and satisfied the following conditions: a concentration of polyvalent cations is less than 0.2 mmol/l; and a concentration of sodium ions is 0.3 mmol/l or more and less than 500 mmol/l.

(Rinsing water)

A:

Functional water, which is prepared by treating tap water supplied in Matsuyama City, Ehime Japan, with a cation exchange resin, and satisfies the following conditions: a concentration of polyvalent cations is less than 0.2 mmol/l; and a concentration of sodium ions is 0.3 mmol/l or more and less than 500 mmol/l.

B:

Tap water supplied in Matsuyama City, Ehime Japan.
The residual amount of a surfactant shown in Table 4 is equivalent to an amount of the soap, which is calculated based on an amount of sodium laurate quantified by gas chromatography.

Table 4

| | Examinee | Cleaning agent | Rinsing water | Residual amount of surfactant ($\mu$g/cm$^2$) |
|---|---|---|---|---|
| Example 11 | A | A | A | Below detection limit |
| | B | A | A | Below detection limit |
| | C | A | A | Below detection limit |
| Example 12 | A | A | B | 22.8 |
| | B | A | B | 29.9 |
| | C | A | B | 32.2 |
| Example 13 | A | B | A | Below detection limit |
| | B | B | A | Below detection limit |
| | C | B | A | Below detection limit |
| Example 14 | A | B | B | 19.5 |
| | B | B | B | 10.5 |
| | C | B | B | 13.2 |

Example 15

A soap (trade name of "Nantaro Soap" manufactured by Miura Co., Ltd.) was dissolved in water to prepare soapy water (cleaning agent) of 0.01% by weight concentration. The water used herein was the functional water obtained by treating tap water supplied in Matsuyama City, Ehime Japan, with a cation exchange resin. The water satisfied the conditions that a concentration of polyvalent cation is less than 0.2 mmol/l and a concentration of sodium ions is 0.3 mmol/l or more and less than 500 mmol/l.

Example 16

Soapy water (cleaning agent) was prepared in the same manner as in Example 15, except for changing the concentration to 0.05% by weight.

Comparative Example 11

A soap (trade name of "Nantaro Soap" manufactured by Miura Co., Ltd.) was dissolved in tap water supplied in Matsuyama City, Ehime Japan to prepare soapy water of 0.01% by weight concentration.

Comparative Example 12

Soapy water was prepared in the same manner as in Comparative Example 11, except for changing the concentration to 0.05% by weight.

Evaluation 5

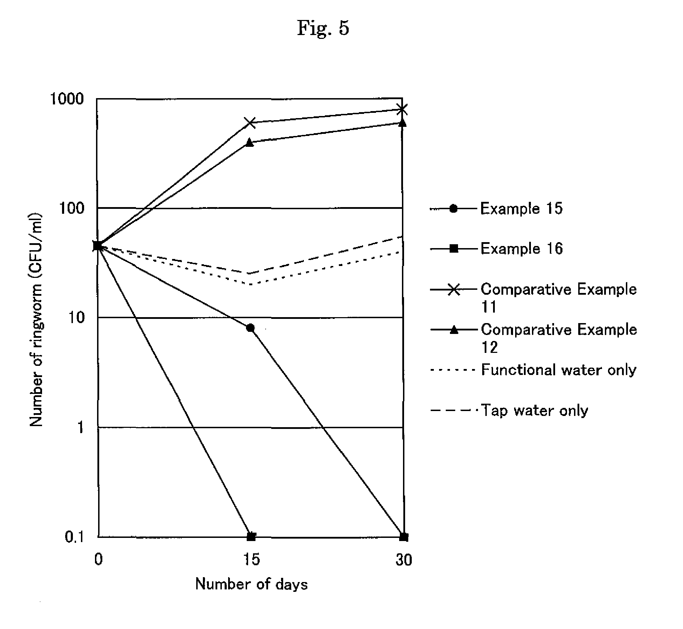

Ringworm (Trichophyton rubrum NBRC32409) was added to the soapy water prepared in Examples 15 and 16 and Comparative Examples 11 and 12 in an amount of about 30 CFU/ml, which was allowed to stand at a temperature of 25°C for 30 days. The unit "CFU" stands for "colony forming unit". During this time, the diachronic change in the number of ringworm in the soapy water was measured everyday. The results are shown in Fig. 5. For reference, Fig. 5 also shows diachronic changes in the number of ringworm in case of adding ringworm to the functional water alone used in Examples 15 and 16 (denoted as "functional water only" in Fig. 5), and in case of adding ringworm to the tap water alone used in Comparative Examples 11 and 12 (denoted as "tap water only" in Fig. 5). The measurement of the number of ringworm was conducted as follows.

Soapy water (50 ml) containing ringworm in a 100 ml Erlenmeyer flask was stirred at a rate of 10, 000 rpm for 5 minutes using a homogenizer (trade name of "Ace Homogenizer AM-3 Nihonseiki Kaisha LTD.) to dissociate the ringworm, and then it was subjected to ultrasonic waves. A sample of 100 μl was taken out from the soapy water and used without being diluted. The sample was cultured at 25°C for 5 days using a PDA (Potato Dextrose Agar) plate medium containing chloramphenicol, and the number of the growing ringworm colonies was counted by visual observation. Based on the result, the number of ringworm contained in the soapy water was calculated using the following equation (5)

$$\text{Number of ringworm (CFU/ml)} =$$
$$\text{Number of ringworm colonies} \times 10 \qquad (5)$$

According to Fig. 5, the soapy water (cleaning agent) used in Examples 15 and 16 excels in sterilizing effect against ringworm.

Examples 17 to 25

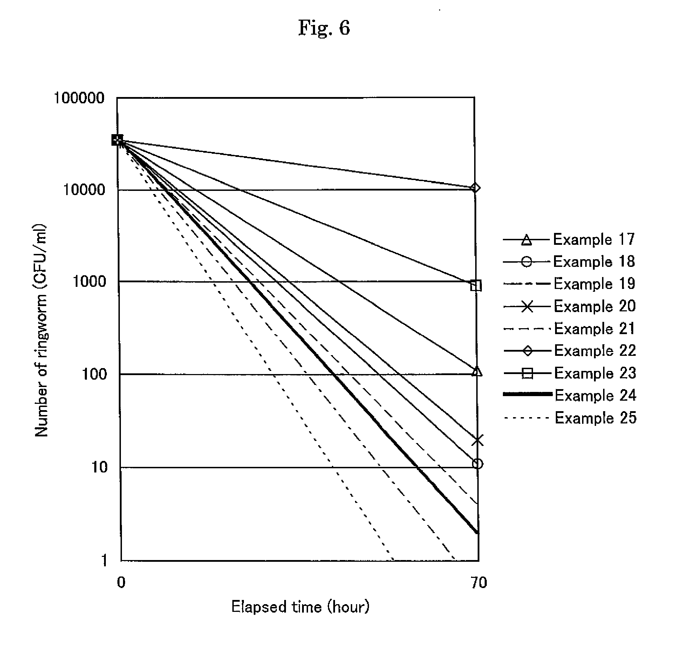

A fatty acid sodium salt shown in Table 5 was added and dissolved in the functional water, which was obtained by treating tap water supplied in Matsuyama City, Ehime Japan, with a cation exchange resin, to prepare a 5 mM aqueous solution of fatty acid sodium salt (cleaning agent). Ringworm (Trichophyton mentagrophytes) was added to the aqueous solution of fatty acid sodium salt in an amount of about $2 \times 10^4$ CFU/ml, which was shaken at 35°C, and the diachronic change in the number of the ringworm was measured over the following 70 hours. The number of the ringworm was measured as follows. After shaking the aqueous solution of fatty acid sodium salt containing ringworm, a sample of 100 µl was taken therefrom and properly diluted. The diluted sample was cultured at 25°C for 5 days using a PDA (Potato Dextrose Agar) plate medium containing chloramphenicol, and the number of the growing ringworm colonies was counted by visual observation. Based on the result, the number of the ringworm contained in the cleaning agent was calculated using the following equation (6). The results are shown in Fig. 6.

```
Number of ringworm (CFU/ml) =

    Number of ringworm colonies × Dilution rate × 10
```

$$(6)$$

Table 5

| Example | Fatty acid sodium salt | | |
|---------|------------------------|---------------|-------------------------------------|
|         | Name                   | Carbon number | Number of carbon-carbon double bond |
| 17      | Sodium laurate         | 12            | 0                                   |
| 18      | Sodium myristate       | 14            | 0                                   |
| 19      | Sodium myristolate     | 14            | 1                                   |
| 20      | Sodium palmitate       | 16            | 0                                   |
| 21      | Sodium palmitolate     | 16            | 1                                   |
| 22      | Sodium stearate        | 18            | 0                                   |
| 23      | Sodium oleate          | 18            | 1                                   |
| 24      | Sodium linoleate       | 18            | 2                                   |
| 25      | Sodium linolenate      | 18            | 3                                   |

According to Fig. 6, it is found that an aqueous solution of a fatty acid sodium salt exhibits high sterilizing ability particularly when a fatty acid sodium salt having 12 to 16 carbon atoms is used. When carbon numbers of the fatty acid sodium salts are identical, unsaturated fatty acid sodium salts, particularly those having many carbon-carbon double bonds, exhibit higher sterilizing power.

Example 26

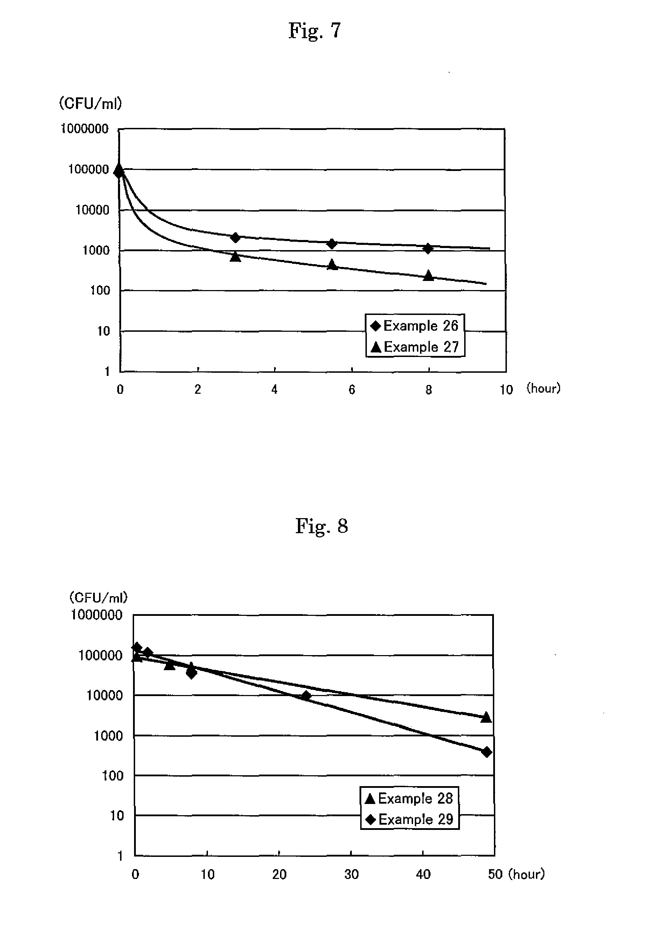

A sodium myristate salt was added and dissolved in functional water, which was obtained by treating tap water supplied in Matsuyama City, Ehime Japan, with a cation exchange resin, to prepare a cleaning agent of 5 mM concentration. Black mold (Cladosporium sphaerospermum NBRC4460) was added to the cleaning agent in an amount of about $1 \times 10^5$ CFU/ml, which was shaken at 35°C, and then the diachronic change in the number of the black mold spores was measured. The number of the black mold spores was measured as follows. First, the cleaning agent containing the black mold was properly diluted with a sterilized phosphate buffer solution. Then, a sample of 100 µl taken therefrom was cultured at 25°C for 5 days using a PDA (Potato Dextrose Agar) plate medium containing chloramphenicol, and the number of the growing black mold colonies was counted by visual observation. Based on the result, the number of the black mold contained in the cleaning agent was calculated using the following equation (7). The results are shown in Fig. 7.

```
Number of black mold spores (CFU/ml) =

       Number of black mold colonies × Dilution rate × 10
```

$$(7)$$

Example 27

The operation was implemented in the same manner as in Example 26, except for using a sodium linolenate salt instead of a sodium myristate salt, and the diachronic change in the number of black mold spores was measured. The results are shown in Fig. 7.

Example 28

A sodium myristate salt was added and dissolved in the functional water, which was obtained by treating tap water supplied in Matsuyama City, Ehime Japan, with a cation exchange resin, to prepare a cleaning agent of 5 mM concentration. Colon bacillus (E. coli NBRC3301) was added to the cleaning agent in an amount of about $1 \times 10^5$ CFU/ml, which was shaken at 35°C, and then the diachronic change in the number of the colon bacillus was measured. The number of the colon bacillus was measured as follows. First, the cleaning agent containing the colon bacillus was properly diluted with a sterilized phosphate buffer solution. Then, a sample of 100 μl taken therefrom was cultured at 35°C for 3 days using a standard agar medium, and the number of the growing colon bacillus colonies was counted by visual observation. Based on the result, the number of the colon bacillus contained in the cleaning agent was calculated using the following equation (8). The results are shown in Fig. 8.

```
Number of colon bacillus (CFU/ml) =

       Number of colon bacillus colonies × Dilution rate × 10
```

$$(8)$$

Example 29

The operation was implemented in the same manner as in Example 28, except for using a sodium linolenate salt instead of a sodium myristate salt, and the diachronic change in the number of colon bacillus was measured. The results are shown in Fig. 8.

Example 30

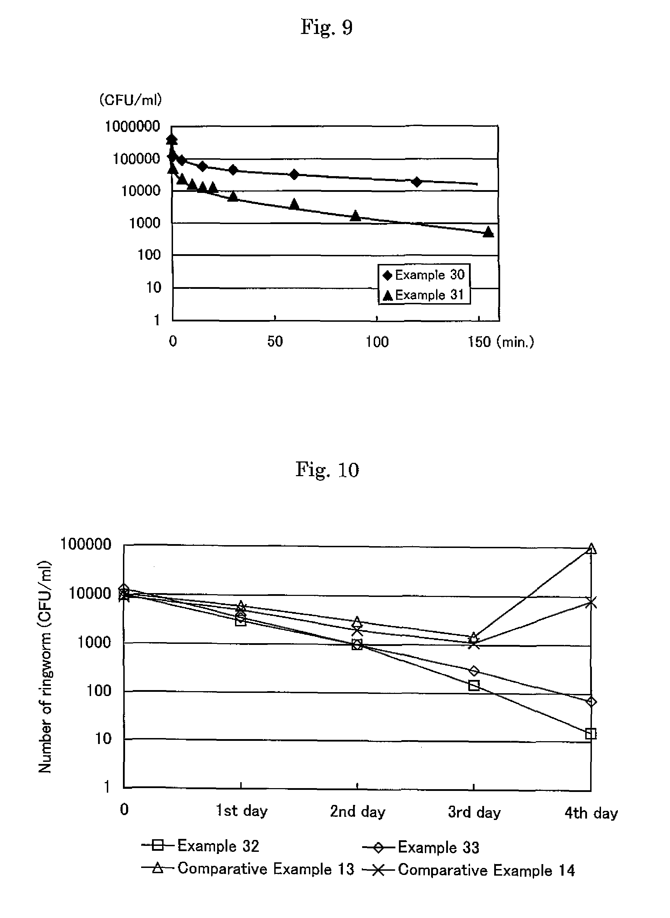

A sodium myristate salt was added and dissolved in functional water, which was obtained by treating tap water supplied in Matsuyama City, Ehime Japan, with a cation exchange resin, to prepare a cleaning agent of 5 mM concentration. Staphylococcus aureus bacteria (S. aureus NBRC13276) was added to the cleaning agent in an amount of about $1 \times 10^5$ CFU/ml, which was shaken at 35°C, and then the diachronic change in the number of the staphylococcus aureus bacteria was measured. The number of the staphylococcus aureus bacteria was measured as follows. First, the cleaning agent containing the staphylococcus aureus bacteria was properly diluted with a sterilized phosphate buffer solution. Then, a sample of 100 μl taken therefrom was cultured at 35°C for 3 days using a standard agar medium, and the number of the growing staphylococcus aureus bacterial colonies was counted by visual observation. Based on the result, the number of the staphylococcus aureus bacteria (S. aureus bacteria) contained in the cleaning agent was calculated using the following equation (9). The results are shown in Fig. 9.

$$\text{Number of S. aureus bacteria (CFU/ml)} =$$

$$\text{Number of S. aureus bacterial colonies} \times \text{Dilution rate} \times 10$$

(9)

Example 31

The operation was implemented in the same manner as in Example 30, except for using a sodium linolenate salt instead of a sodium myristate salt, and the diachronic change in the number of staphylococcus aureus bacteria was measured. The results are shown in Fig. 9.

Example 32

A sodium linoleate salt was added and dissolved in functional water, which was obtained by treating tap water supplied in Matsuyama City, Ehime Japan, with a cation exchange resin, to prepare a cleaning agent of 1 mM concentration. Ringworm (Trichophyton mentagrophytes) was added to the cleaning agent in an amount of about $1 \times 10^4$ CFU/ml, which was shaken at 35°C, and the diachronic change in the number of the ringworm was measured. The number of the ringworm was measured in the same manner as in Examples 17 to 25. The results are shown in Fig. 10.

Example 33

The operation was implemented in the same manner as in Example 32, except for using a sodium linolenate salt instead of a sodium linoleate salt, and the diachronic change in the number of ringworm was measured. The results are shown in Fig. 10.

Comparative Example 13

The operation was implemented in the same manner as in Example 32, except for using tap water of Matsuyama City, Ehime Japan, instead of the functional water, and the diachronic change in the number of ringworm was measured. The results are shown in Fig. 10.

Comparative Example 14

The operation was implemented in the same manner as in Example 33, except for using tap water of Matsuyama City, Ehime Japan, instead of the functional water, and the diachronic change in the number of ringworm was measured. The results are shown in Fig. 10.

Example 34

The skin state of 8 examinees who took bath everyday for 4 weeks using a cleaning agent was studied. The examinees were female aging from 30 to 47 year old (average age: 36.9 years old). The cleaning agents used herein were prepared by dissolving detergents shown in Table 6 in the functional water. The functional water was obtained by treating tap water with a cation exchange resin, and satisfied the conditions that a concentration of polyvalent cation is less than 0.2 mmol/l and a concentration of sodium ions is 0.3 mmol/l or more and less than 500 mmol/l.

Table 6

| Examinee | | Detergent | | |
|---|---|---|---|---|
| Number | Age | Kind | Name of manufacturer | Trade name |
| 1 | 42 | Body shampoo | Unilever Japan | Dove body wash |
| 2 | 37 | Body shampoo Body shampoo | Shiseido Co., Ltd. | Kuyura |

(continued)

| Examinee | | Detergent | | |
|---|---|---|---|---|
| Number | Age | Kind | Name of manufacturer | Trade name |
| 3 | 36 | Body shampoo | Kanebo Cosmetic Inc. | Naive body soap N, moisture milk |
| 4 | 47 | Body shampoo | Amway Japan Ltd. | Satinique body shampoo |
| 5 | 34 | Soap | Kanebo Cosmetic Inc. | Silk soap Silk soap |
| 6 | 30 | Body shampoo | Unilever Japan | Dove body care wash |
| 7 | 30 | Body shampoo | Kao Corp. | Biore |
| 8 | 39 | Soap | Yugen Kaisha Yugen Kaisha Neba Juku | Soap Seseragi |

While taking a bath, the examinees washed their bodies with the cleaning agent, rinsed the cleaning agent off with the above functional water alone, and soaked in a bathtub filled with the heated functional water alone.

<Evaluation A>

Each item of stratum corneum moisture content, skin elasticity and texture density was measured on every examinee by the following methods. The mean value of each item was compared among the day when the test was started, 2 weeks after test start and 4 weeks after test start.

(Stratum corneum moisture content)

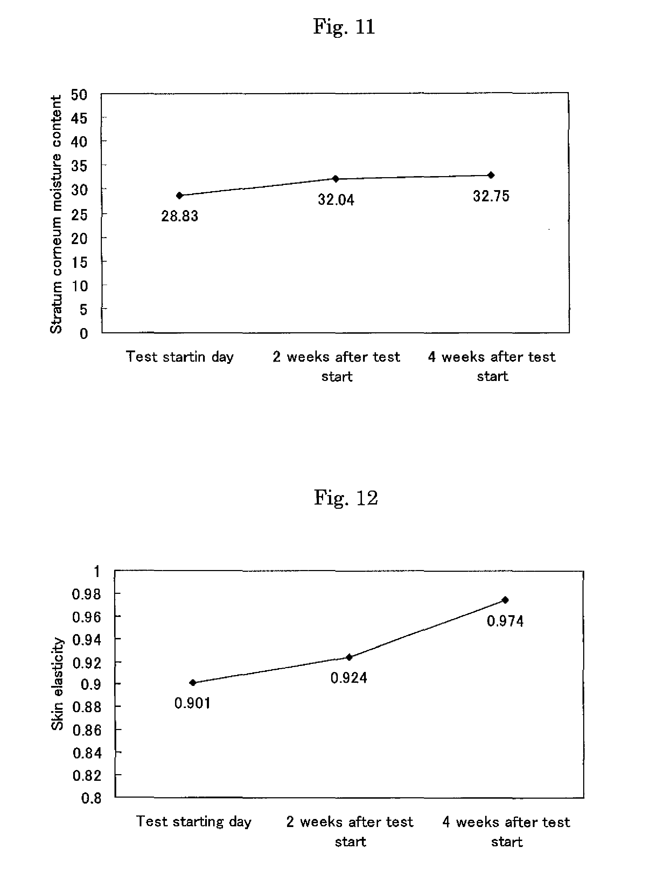

An electric characteristic of skin, that is, capacitance of skin surface was measured to determine stratum corneum moisture content with a measuring device of stratum corneum moisture content (trade name of "Corneometer CM825" manufactured by Courage + Khazaka Electronic GmbH). The measurement was conducted 3 times, and the mean value was served as the measurement value. The results are shown in Fig. 11.

(Skin elasticity)

Skin electricity was measured using a measuring device of skin elasticity (trade name of "Cutometer SEM575" manufactured by Courage + Khazaka Electronic GmbH), which is equipped with a measurement probe having a suction hole, that is, a negative pressure aspirator, a pressure sensor, and a computer for operation thereof and data processing. In this measurement, when the probe is guided to a skin surface to start the measurement, a negative pressure is applied to the probe suction hole and thus the skin inside the suction hole is sucked in. Then, a height of the sucked skin was measured with a light sensor contactlessly without generation of friction or mechanical action. The measurement was conducted 4 times, and the mean value was served as the measurement value. The results are shown in Fig. 12.

(Texture density)

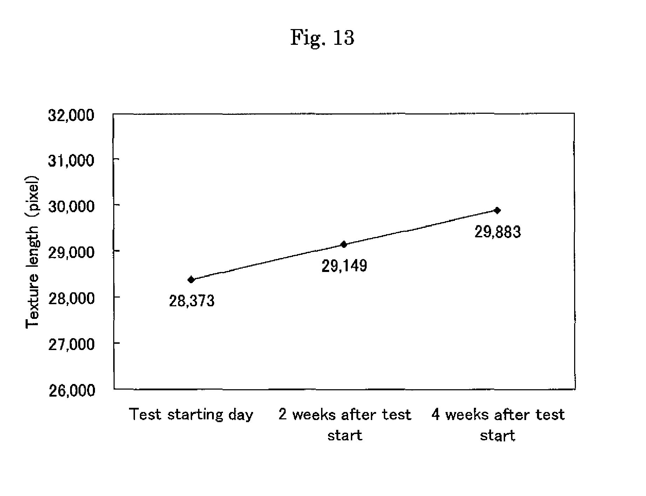

Texture density was measured by applying image analysis software (trade name of "Skin Analysis Software" manufactured by Inforward Inc.) for the purpose of evaluating a state of the texture distribution to a color image of skin obtained by a device with a trade name of "microscope VI-27" manufactured of FineOpt Co., Ltd. Specifically, image data processing was applied by the above image analysis software to a color image obtained so as to emphasize and extract the texture region, and a total length of the texture region (unit: pixel) was calculated. The results are shown in Fig. 13. Texture density denotes a percentage (%) of a texture length in an area of the obtained color image, and is therefore substantially in a relation of "texture length (pixel)" = "texture density (%)".

<Evaluation B>

On the same day when each item in Evaluation A was measured, drying condition of skin and existence or nonexistence of scale were evaluated on every examinee, according to a diagnosis by a doctor. The results are shown in Table 7. In Table 7, evaluation criteria on each item are as follows.

(Drying condition)

None: The symptom is not observed.
Minor: The symptom is slightly observed.
Mild: The symptom is somewhat observed.
Moderate: The symptom is clearly observed.
Severe: The symptom is remarkably observed.

(Existence or nonexistence of scale)

None: The symptom is not observed.
Minor: The symptom is slightly observed.
Mild: The symptom is somewhat observed.
Moderate: The symptom is clearly observed.
Severe: The symptom is remarkably observed.

Table 7

| | | None | Minor | Mild | Moderate | Severe |
|---|---|---|---|---|---|---|
| Drying condition | Test starting day | 0 | 1 | 5 | 2 | 0 |
| | Two weeks after test start | 0 | 5 | 3 | 0 | 0 |
| | Four weeks after test start | 0 | 7 | 1 | 0 | 0 |
| Existence or nonexistence of scale | Test starting day | 1 | 6 | 1 | 0 | 0 |
| | Two weeks after test start | 1 | 7 | 0 | 0 | 0 |
| | Four weeks after test start | 7 | 0 | 1 | 0 | 0 |

<Evaluation C>

On the same day when each item in Evaluation A was measured, itchy feeling of skin based on the examinees' own complaint was evaluated according to a diagnosis by a doctor. The results are shown in Table 8. In Table 8, evaluation criteria on itchy feeling are as follows.

None: The symptom is not observed.
Minor: The symptom is slightly observed.
Mild: The symptom is somewhat observed.
Moderate: The symptom is clearly observed.
Severe: The symptom is remarkably observed.

Table 8

| | | None | Minor | Mild | Moderate | Severe |
|---|---|---|---|---|---|---|
| Itchy feeling | Test starting day | 3 | 1 | 3 | 1 | 0 |
| | Two weeks after test start | 6 | 2 | 0 | 0 | 0 |
| | Four weeks after test start | 8 | 0 | 0 | 0 | 0 |

<Evaluation D>

The results of questionnaire relating to self-evaluation on the examinees' own skin are shown in Table 9, which was implemented to each examinee before and after the test.

Table 9

| | | Feel | Somewhat feel | Not Normal | quite feel | Not feel |
|---|---|---|---|---|---|---|
| There is a taut feeling after taking a bath | Before test start | 3 | 2 | - | 1 | 2 |
| | After test termination | 0 | 0 | - | 4 | 4 |
| Skin is dry | Before test start | 4 | 2 | 2 | 0 | 0 |
| | After test termination | 1 | 3 | 1 | 2 | 1 |
| Skin is smooth | Before test start | 0 | 3 | 2 | 1 | 2 |
| | After test termination | 2 | 4 | 1 | 0 | 1 |
| Skin has tightness and elasticity | Before test start | 0 | 0 | 5 | 1 | 2 |
| | After test termination | 0 | 3 | 4 | 0 | 1 |
| Skin texture is finer | Before test start | 0 | 2 | 4 | 1 | 1 |
| | After test termination | 0 | 5 | 2 | 1 | 0 |

The results of Evaluations A to D indicate that in case of taking a bath continuously using the cleaning agent, skin health condition is hardly damaged, and rather it tends to improve.

The present invention can be practiced in other various forms without departing from the spirit and principal features thereof. In view of this, the embodiments or examples described above merely serve as exemplification in every respect and should not be construed restrictively. A scope of the present invention is defined by claims, and is by no means bound by the text of the specification. Furthermore, all modifications and alternations belonging to the equivalent scope of the claims fall within the scope of the present invention.

**Claims**

1. A cleaning agent comprising,
   water from which polyvalent cations are removed and to which a sodium ion is added, and
   a surfactant.

2. The cleaning agent according to claim 1, which is used for kitchens.

3. The cleaning agent according to claim 1, which is used for tableware.

4. The cleaning agent according to claim 1, which is used for food.

5. The cleaning agent according to claim 1, which is used for washstands.

6. The cleaning agent according to claim 1, which is used for bathrooms.

7. The cleaning agent according to claim 1, which is used for toilets.

8. The cleaning agent according to claim 1, which is used for vehicles.

9. The cleaning agent according to claim 1, which is used for clothes.

10. The cleaning agent according to claim 1, which is used for skin.

11. The cleaning agent according to any of claims 1 to 10, wherein the surfactant is a fatty acid salt.

12. The cleaning agent according to claim 11, wherein the fatty acid salt is an unsaturated fatty acid salt.

13. The cleaning agent according to claim 12, wherein the unsaturated fatty acid salt is at least one selected from the group consisting of linoleate, linolenate, myristolate and palmitolate.

Fig. 1

Fig. 2

## Fig. 3

## Fig. 4

Fig. 5

Fig. 6

# Fig. 7

# Fig. 8

# Fig. 9

(CFU/ml)

Legend: ◆ Example 30, ▲ Example 31

X-axis: 0, 50, 100, 150 (min.)

# Fig. 10

Y-axis: Number of ringworm (CFU/ml)

X-axis: 0, 1st day, 2nd day, 3rd day, 4th day

Legend: —☐— Example 32   —◇— Example 33
—△— Comparative Example 13   —✕— Comparative Example 14

Fig. 11

Fig. 12

Fig. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10508901 A